# EUROPEAN PATENT APPLICATION

(11) **EP 4 272 760 A1**
(43) Date of publication of application: **08.11.2023**
(21) Application number: 21912892.3
(22) Date of filing: 04.06.2021
(51) Int. Cl.: A61K 45/06, A61K 31/7135, A61K 31/095, A61K 31/145, A61P 35/00

(54) **ANTI-CANCER COMPOSITION CONTAINING AURANOFIN AND SULFHYDRYL COMPOUND AND USE THEREOF**

(30) Priority: 30.12.2020 CN 202011617321
(71) Applicant: Sun Yat-Sen University, Guangzhou, Guangdong 510275 (CN)
(72) Inventor: ZOU, Taotao, Guangzhou, Guangdong 510275 (CN); WANG, Yuan, Guangzhou, Guangdong 510275 (CN); XIONG, Xiaolin, Guangzhou, Guangdong 510275 (CN); CHEN, Xinzi, Guangzhou, Guangdong 510275 (CN)
(74) Representative: Gassner, Birgitta
(86) International application number: PCT/CN2021/098230
(87) International publication number: WO 2022/142131

(57) **Abstract**

Disclosed is an anticancer composition containing auranofin and a mercapto compound and the use thereof. The composition contains auranofin and the mercapto compound. By the combined use of auranofin and the mercapto compound, the problem that auranofin cannot effectively inhibit the proliferation of cancer cells under normal physiological condition is solved. When combined with the mercapto compound, auranofin can inhibit tumor growth well, indicating that the combination of auranofin and the mercapto compound can be used as an anticancer drug composition. Moreover, the uptake of auranofin in tumor tissues also can be adjusted by regulating the type of mercapto compound, and thus, providing better selectivity in vivo anti-cancer.

## Description

### TECHNICAL FIELD

The present disclosure belongs to the field of pharmaceutical compositions, and in particular relates to an anti-cancer composition containing auranofin and a mercapto compound and use thereof.

### BACKGROUND

Metal drugs represented by cisplatin are important clinical anticancer drugs, but a number of tumors have intrinsic resistance to platinum drugs or develop resistance as the dosage increases, and patients are prone to have strong adverse reactions (such as bone marrow suppression, gastrointestinal reactions, renal toxicity, neurotoxicity, allergic reactions, electrolyte disturbances and hepatic dysfunction) during use. Therefore, the practical application of the metal drugs is greatly limited.

Auranofin is an oral anti-rheumatic drug, which is mainly used for treating active rheumatoid arthritis. In earlier studies, auranofin can effectively prolong the life span of mice inoculated with acute lymphocytic leukemia cell P3 88. However, follow-up studies have shown that in several solid tumor models, auranofin (administered via intraperitoneal injection) could neither prolong the life span of mice nor inhibit the growth of tumors.

Therefore, it is necessary to further verify and explore whether auranofin has stable anticancer activity and the mechanism of the anticancer effect thereof. Moreover, the combined anticancer effect of auranofin and other drugs has not been disclosed, the mechanism of its anticancer effect when auranofin is combined with other drugs is of great significance for the research and development of anticancer drugs.

### SUMMARY

An object of the present disclosure is to provide a composition.

Another object of the present disclosure is to provide a drug.

Another object of the present disclosure is to provide a cell proliferation inhibitor.

Another object of the present disclosure is to provide use of the composition in the preparation of an anticancer drug.

Another object of the present disclosure is to provide use of the composition in the preparation of a cell proliferation inhibitor.

Another object of the present disclosure is to provide use of the composition in the preparation of a Thioredoxin Reductase (TrxR) inhibitor.

Another object of the present disclosure is to provide use of a mercapto compound serving as a synergist of auranofin in the preparation of a TrxR inhibitor.

The technical solution used in the present disclosure is:
in a first aspect of the present disclosure, a composition containing auranofin and a mercapto compound is provided.

Since auranofin will quickly bind to human serum albumin (HSA) after entering the human body and thiol proteins on cell membranes will bind to gold ions, the effective uptake of the gold ions is reduced, and the cytotoxicity of auranofin is reduced, preventing the auranofin from exerting an effective cancer inhibition effect. The composition of the present disclosure contains the mercapto compound, which can inhibit the binding of auranofin to mercapto groups in blood proteins in vivo, so that the cellular uptake of auranofin is enhanced, and the in vivo anticancer activity of auranofin is further regulated.

By the advantages of the ligand exchange property of auranofin and the high affinity to the mercapto group, the in vivo anticancer activity of auranofin can be restored by the addition of a specific mercapto compound, which demonstrates that auranofin has higher anticancer activity when combined with lipophilic small molecules containing mercapto group.

A mercapto compound comprises a compound with a structure as shown in Formula I or a thiolate:

R-SH (Formula I)

wherein, R is selected from any group;

further, the thiolate is a metal thiolate.

Furthermore, the mercapto compound is one or more of β-mercaptoethanol, mercaptoethanamine, methylthioimidazole, propylthiouracil, dithiothreitol, N-acetylcysteine, glutathione, sodium thiosulfate, sodium thiophosphate, sodium diethyldithiocarbamate or 1-thio-β-D-glucose tetraacetate.

Definitely, depending on actual demands, an acetylated mercapto glycoside ligand of auranofin may also include mercaptomannose, mercaptoglucose, mercaptosucrose or other mercaptocarbohydrate-modified auranofin ligands.

Furthermore, the added amount of the mercapto compound is within the range of non-toxic dose.

In a second aspect of the present disclosure, a drug containing the above composition is provided.

The drug of the present disclosure contains the mercapto compound, which can inhibit the binding of auranofin to mercapto groups in blood proteins in vivo, so that the cellular uptake of auranofin is enhanced, and the in vivo anticancer activity of auranofin is further regulated.

In a third aspect of the present disclosure, a cell proliferation inhibitor containing the above composition is provided.

The inventor found that the tumor growth could not be inhibited when auranofin or the mercapto compound was administered alone; however, a strong inhibitory effect on tumor growth was shown when auranofin and the mercapto compound were used in combination.

Further, the cells include colon cancer cells, liver cancer cells, lung cancer cells, gastric cancer cells, cervical cancer cells and breast cancer cells.

Furthermore, the colon cancer cells are human colon cancer cells.

Furthermore, the liver cancer cells are human liver cancer cells.

Furthermore, the lung cancer cells are human lung cancer cells.

Furthermore, the breast cancer cells are human breast cancer cells.

In a fourth aspect of the present disclosure, use of the composition in the preparation of an anticancer activity drug is provided.

Further, the cancers include colon cancer, liver cancer, lung cancer, gastric cancer cells, cervical cancer cells and breast cancer.

In a fifth aspect of the present disclosure, use of the composition in the preparation of a cell proliferation inhibitor is provided.

Further, the cells are cancer cells; the cancer cells include colon cancer cells, liver cancer cells, lung cancer cells, gastric cancer cells, cervical cancer cells and breast cancer cells.

In a sixth aspect of the present disclosure, use of the composition in the preparation of a TrxR inhibitor is provided.

In a seventh aspect of the present disclosure, use of a mercapto compound serving as a synergist of auranofin in the preparation of a TrxR inhibitor is provided.

The mercapto compound is a compound containing mercapto.

Further, the mercapto compound comprises one or more of β-mercaptoethanol, mercaptoethanamine, methylthioimidazole, propylthiouracil, dithiothreitol, N-acetylcysteine, glutathione, sodium thiosulfate, sodium thiophosphate, sodium diethyldithiocarbamate or 1-thio-β-D-glucose tetraacetate.

The present disclosure has the following beneficial effects.

In the disclosure, auranofin cannot effectively inhibit the proliferation of HCT116 human colon cancer cells, A549 human lung cancer cells, MCF-7 human breast cell cancer cells and HepG-2 human liver cancer cells (with a IC₅₀ value greater than 30 µM) under the normal physiological condition. However, when a non-toxic dose of mercapto compound (β-mercaptoethanol, 1-thio-β-D-glucose tetraacetate, etc.) is added, auranofin shows an excellent inhibitory effect on the above 4 cancer cells, including inhibition of TrxR and tumor cell proliferation. The experiments in a mouse tumor model also indicates that auranofin can inhibit the tumor growth well when combined with the mercapto compound, indicating that the combination of auranofin and the mercapto compound can be used as an anticancer drug composition. Moreover, the uptake of auranofin in tumor tissues also can be adjusted by regulating the type of mercapto compound, and thus, providing better selectivity in vivo anti-cancer.

### BRIEF DESCRIPTION OF THE DRAWINGS

Fig.1 shows the inhibitory effect of auranofin in combination with various mercapto compounds on TrxR activity;
Fig.2 shows the effect of auranofin in combination with 1-thio-β-D-glucose tetraacetate (TGTA) on tumor volume in a mouse tumor model;
Fig.3 shows the effect of auranofin in combination with TGTA on weight of mice in a mouse tumor model;
Fig.4 shows the actual effect of auranofin in combination with TGTA in a mouse tumor model, where A is the image of a tumor and B is the image of a mouse;
Fig.5 shows the effect of auranofin in combination with β-mercaptoethanol on tumor volume in a mouse tumor model;
Fig.6 shows the effect of auranofin in combination with β-mercaptoethanol on weight of mice in a mouse tumor model; and
Fig.7 shows an image of auranofin in combination with β-mercaptoethanol on a tumor in a mouse tumor model.

### DETAILED DESCRIPTION

To better clarify the inventive purpose, technical solution and technical effect of the present disclosure, the present disclosure is further described in detail in conjunction with specific embodiments. It should be understood that the specific embodiments described in the present description are merely intended to explain the present disclosure, but not as a limit to the present disclosure.

Unless otherwise specified, the experimental materials and reagents used are all conventional consumables and reagents that are commercially available.

### Inhibitory effect of auranofin in combination with various mercapto compounds on the activity of various cancer cells

### (1) Culture of cancer cells

A flask with the cancer cells required for the experiment, with an original culture solution discarded, was washed for three times with 2 mL of PBS, 1 mL of pancreatin was added for digestion for 30 s, and 2 mL of a medium was added to terminate the digestion. The cells were transferred to a centrifuge tube and centrifuged at 1000 rpm for 3 min, the supernatant was discarded, and 2 mL of a medium was added and blew 10 times to be mixed uniformly. 10 µL of a cell suspension was taken for cell counting, and spread on a 96-well plate, with 5000 cells per well and 3 duplications for each compound, and then the number of cells and cell wells was calculated. The amount of required cell suspension and the number of cells for medium dilution were calculated by cell counting. 100 µL of medium was plated with 5000 cells per well, pipetted and mixed uniformly, and the cells were inoculated using a volley, placed in an incubator for culture, and marked.

(2) The cells cultured in step (1) was taken, and discarded with the original medium. Auranofin mother liquor was diluted to a diluent with final concentrations of 0, 0.78125, 1.5625, 3.125, 6.25, 12.5, 25, 50 and 100 µM, and mixed uniformly. A volley (using 3 wells) was used to draw 100 µL of dilution per well, a 96-well plate was tilted, diluents with each concentration were added in sequence to the corresponding 3 marked wells carefully to prevent poking the cells, blown and sucked about 3 times to be mixed uniformly, marked, shaken for 3 times, and put into a CO₂ incubator for culture. The above steps were repeated for each auranofin diluent.

After auranofin was added for culture for 1 hour, the mother liquor of the mercapto compound was diluted into 1 M and mixed uniformly. A volley (using 3 wells) was used to suck 20 µL of diluent per well, the 96-well plate was tilted, the diluents of different mercapto compounds were added in sequence to the corresponding 3 marked wells carefully to prevent poking the cells, blown and sucked about 3 times to be mixed uniformly, and put back to the incubator to continue culture.

### (3) Detection of the inhibitory effect on cancer cell proliferation by an MTT colorimetric method

After different mercapto compounds were added to cells for 24 h, 20 µL of MTT (with a concentration of 5 mg/mL in PBS) per well was added to the 96-well plate by a volley; and incubation was carried out at 37° C for 4 h. At the end of incubation, the MTT mixture was sucked out, and DMSO was added at 130 µL/well using a volley. The cells were shaken for 10 min, after Formazan was fully dissolved, the absorbance was detected at a wavelength of 490 nm with a microplate reader, the survival rate of the cells at various concentrations of drugs was calculated as required to draw a scatter diagram, and the IC₅₀ values of auranofin in combination with various compounds (containing 10% fetal bovine serum (FBS) or 40 mg/mL Bovine albumin (BSA)) on the proliferation of cancer cells are as shown in Tables 1 and 2, where auranofin alone (without BSA/FBS) was used as the control. The IC₅₀ values of auranofin alone are shown in Table 3.

**Table 1. Inhibitory effect of auranofin in combination with various mercapto compounds (with 10% FBS) on proliferation of cancer cells**

| Group | Auranofin | β-mercaptoethanol | Sodium diethyldithiocarbamate (DEDT) | IC₅₀ | | | |
|---|---|---|---|---|---|---|---|
| | | | | HCT116 Human colon cancer cells | HepG-2 Human liver cancer cells | A549 Human lung cancer cells | MCF-7 Human breast cell cancer cells |
| 1 | + | - | - | 3.4 | 3.0 | 12.9 | 5.7 |
| 2 | + | + | - | <0.625 | <0.625 | 4.0 | 0.9 |
| 3 | + | - | + | <0.625 | <0.625 | 4.3 | - |

| | | | | | | | |
|---|---|---|---|---|---|---|---|
| Where "+" means addition. | | | | | | | |

**Table 2. Inhibitory effect of auranofin in combination with 200 µM of various compounds (containing 40 mg/mL BSA on proliferation of cancer cells**

| Gro up | Aurano fin | β-mercaptoeth anol | Mercaptoethyla mine | TGT A | IC₅₀ | | | | |
|---|---|---|---|---|---|---|---|---|---|
| | | | | | HCT1 16 Huma n colon cancer cells | Hep G-2 Hum an liver canc er cells | A549 Hum an lung canc er cells | MCF -7 Hum an breas t cell canc er cells | HCT-8 Human colon adenocarcin oma cells |
| 1 | + | - | - | - | 30.0 | 16.6 | 70.5 | 39.7 | 33.2 |
| 2 | + | + | - | - | 5.8 | 2.3 | 32.1 | 19.6 | 15.5 |
| 3 | + | - | + | - | 15.3 | 9.8 | 35.7 | - | 15.9 |
| 4 | + | - | - | + | <1.56 | <1.5 6 | - | - | - |

| | | | | | | | | | |
|---|---|---|---|---|---|---|---|---|---|
| Where "+" means addition. | | | | | | | | | |

**Table 3. Inhibitory effect of auranofin alone (without BSA/FBS) on proliferation of cancer cells**

| | HCT116 Human colon cancer cells | HepG2 Human liver cancer cells | A549 Human lung cancer cells | MCF-7 Human breast cell cancer cells |
|---|---|---|---|---|
| IC₅₀ | 1.0 | 1.3 | 4.2 | 3.4 |

| | | | | |
|---|---|---|---|---|
| Where "+" means addition. | | | | |

According to the results of Table 1-2, the killing effect of auranofin in combination with various compounds (with 30% FBS) on HCT116 human colon cancer cells and the killing effect of the auranofin-TGTA (50 µM) in combination with other cancer cells (lung cancer, colon cancer, breast cancer, liver cancer, cervical cancer and gastric cancer cells) were further detected by using the same method as the above.

The results are shown in Tables 4 and 5.

**Table 4. Killing effect of auranofin in combination with various compounds (with 30% FBS) on HCT116 human colon cancer cells**

| Compounds | Cell survival rate (%) | | Cell survival rate after addition of 10 µM auranofin (%) | |
|---|---|---|---|---|
| Auranofin | | - | | 75 ± 5 |
| 50 µM TGTA | | 97 ± 5 | | 18 ± 2 |
| 50 µM β-mercaptoethanol | | 96 ± 15 | | 55 ± 4 |
| 50 µM mercaptoethylamine | | 105 ± 7 | | 85 ± 3 |
| 50 µM DEDT | | 78 ± 10 | | 0.9 ± 1.6 |
| Methimazole | | 98 ± 9 | | 87 ± 5 |
| Propylthiouracil | | 101 + 6 | | 76 ± 9 |
| Dithiothreitol (DTT) | | 102 ± 4 | | 88 ± 6 |
| N-acetylcysteine (NAC) | | 100 ± 6 | | 77 ± 17 |
| Glutathione (GSH) | | 116 + 6 | | 81 ± 7 |
| sodium thiosulfate | | 105 ± 5 | | 84 ± 8 |
| Sodium thiophosphate hydrate | | 99 ± 8 | | 79 ± 4 |

**Table 5. Effect of auranofin alone, TGTA (50 µM) alone, and auranofin in combination with TGTA on cell survival rate in different cell lines**

| Cell survival rate (%) | Lung cancer | | | colon cancer | breast cancer | Liver cancer | Cervical cancer | Gastric cancer |
|---|---|---|---|---|---|---|---|---|
| | PC9 | PCGR | A549 | HCT116 | MCF-7 | HepG2 | Hela | MGC803 |
| TGTA | 104 + 2 | 101 ± 3 | 104 ± 6 | 97 ± 5 | 99 ± 3 | 104 ± 4 | 87 ± 6 | 104 ± 9 |
| Auranofin | 93 ± 3 | 37 ± 4 | 81 ± 7 | 75 ± 5 | 80 ± 2 | 87 ± 1 | 46 ± 7 | 79 ± 3 |
| Auranofin + TGTA | 2 ± 1 | 11 ± 1 | 15 ± 0.7 | 18 ± 2 | 51 ± 3 | 15 ± 5 | 35 ± 4 | 10 ± 1 |

Results of Table 1-5 indicate that under the normal physiological condition (the addition of FBS or BSA to an empty medium can simulate the high mercapto environment under physiological condition), auranofin has no significant inhibitory effect on HCT116 human colon cancer cells, A549 human lung cancer cells, MCF-7 human breast cell cancer cells, PC9 human lung cancer cells, Hela human cervical cancer cells, MGC803 human gastric cancer and HepG-2 human liver cancer cells. When a non-toxic dose of mercapto compound is added, auranofin shows an excellent inhibitory effect on all 7 cancer cells.

### Mercapto compounds enhanced the inhibitory effect of auranofin on TrxR activity

The detection steps were as follows:
HepG2 cells were used as a test subject.

The cells were inoculated into a 6-well plate at 2 × 10⁵ cell/well and incubated for 24 h.

After the incubation, the cell culture medium was replaced with 30% FBS to simulate the high concentration mercapto environment under the normal physiological condition, and used as a control (without adding any reagents). The experimental group was added with 0.5 µM of auranofin for incubation at 37°C for 30 min, and then added with the mercapto compound (DEDT, β-mercaptoethanol or mercaptoethylamine) for incubation for 30 min (final concentration of DMSO in the system was ≤ 1%). The incubated cells were washed for 3 times with PBS, added with100 µL of icy lysis buffer (containing 50 mM of phosphate buffer with pH 7.4, 1 mM EDTA, and 0.1% Triton-X 100), and then lysed on ice for 5 min to collect the cell lysate.

100 µL of buffer (containing 50 mM of potassium phosphate with pH 7.4, 1 mM EDTA, and 0.2 mM NADPH) was added to the collected cell lysate (10 µg protein), 5,5'-dithiobis (2-nitrobenzoic acid) (DTNB, with a final concentration of 3 mM) was added to initiate a reaction, and the activity of TrxR was obtained on the basis of the increased OD value in 10 minutes (OD detection wavelength was 410 nm). Auranofin alone was the positive control sample, and the blank group was the negative control.

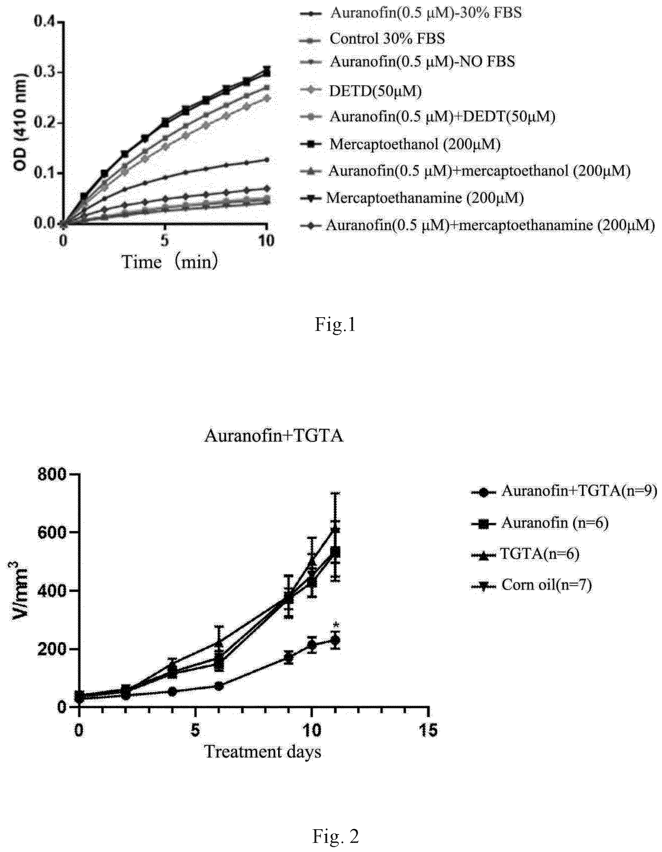

The results are shown in Tables 6 and Figure 1.

**Table 6. Inhibitory effect on activity of TrxR in each experimental group**

| Group | Activity of TrxR |
|---|---|
| Negative control | 100% |
| Positive control (without FBS) | 15.9% |
| Positive control (with 30% FBS) | 63.6% |
| β-mercaptoethanol alone (200 µM) | 127.3% |
| Mercaptoethanamine alone (200 µM) | 118.2% |
| DEDT alone (200 µM) | 88.6% |
| Auranofin in combination with β-mercaptoethanol (200 µM) | 20.5% |
| Auranofin in combination with mercaptoethanamine (200 µM) | 38.6% |
| Auranofin in combination with DEDT (200 µM) | 18.2% |

As show in Table 6, in the absence of FBS (i.e., simulated in vitro conditions), auranofin can inhibit intracellular TrxR activity to 15.9%; however, under the condition of 30% FBS (simulated in vivo condition), the ability of auranofin to inhibit enzyme activity is reduced, and the intracellular TrxR activity is inhibited to 63.6% only. However, when the mercapto compounds are added, even under the condition of 30% FBS, the ability of auranofin to inhibit enzyme activity is greatly improved. Compared with the absence of the mercapto compounds, the ability of auranofin to inhibit enzyme activity is improved by more than 3 times.

### Effect of auranofin in combination with various mercapto compounds on cell uptake of auranofin

The detection steps were as follows:
HepG2 cells were used as a test subject.

The cells were inoculated into a 6-well plate at 2×10⁵ cell/well and incubated for 24 h.

After the incubation, the cell culture medium was replaced with 30% of FBS to stimulate a high-concentration mercapto environment under the normal physiological condition, and used as a control (without adding any reagents). The experimental group was added with 3 µM of auranofin for incubation at 37°C for 10 min and then added with the mercapto compound (DEDT, β-mercaptoethanol or mercaptoethylamine) for incubation for 1 h (final concentration of DMSO in the system was ≤ 1%). After incubation, the cells were immediately washed for 3 times with PBS, 500 µL of ultrapure water was added to each well to break the cells, and after 15 min, the cell lysate was collected.

The collected cell lysate was dissolved by adding aqua regia and diluted with ultrapure water to a proper proportion. Inductively coupled plasma mass spectrometry (ICP-MS) was used for gold detection. The cell uptake rate of 3 µM chynophon without FBS was used as the control.

The results are shown in Table 7.

**Table 7. Effect on the cell uptake rate of auranofin in each experimental group**

| Group | Uptake (mg) | Cell uptake rate (%) |
|---|---|---|
| Control (without FBS) | 728.39 | 100.0 |
| 3 µM auranofin (30% FBS) | 133.15 | 18.3 |
| Auranofin in combination with mercaptoethanamine (200 µM) | 142.31 | 19.5 |
| Auranofin in combination with β-mercaptoethanol (200 µM) | 374.91 | 51.5 |
| Auranofin in combination with DEDT (200 µM) | 321.79 | 44.2 |

Wherein, the above uptake is the amount of gold (mg) in 1g protein in the cell.

As shown in Table 7, in the absence of FBS, with reference to auranofin, the uptake of gold reaches 728 mg/g protein. However, when the concentration of FBS in the medium is increased, the uptake of gold is reduced rapidly; and when the mercapto compound (β-mercaptoethanol, DEDT) is added, the uptake of auranofin is greatly increased. This indicates that the absorption of auranofin in cells is improved after the mercapto compound is added.

### Actual inhibitory effect of auranofin in combination with various mercapto compounds in mouse tumor model

The detection steps were as follows:
Establishment of mouse tumor model: 2 million colon cancer cell HCT1 16 cells suspended in PBS were injected subcutaneously into the dorsal side of 5-7 week-old female BALB/Cann nu (Nude) mice to establish a xenograft model.

When the tumor volume reached about 50 mm³ (3-4 days after tumor inoculation), mice were randomly divided into a control group and a treatment group, the control group was treated with corn oil, the treatment group was intraperitoneally injected with auranofin (3 mg/kg mouse body weight/day) in combination with various mercapto compounds (β-mercaptoethanol or TGTA, 40 mg/kg mouse body weight/day) once a day. When the tumor volume grew to 1000 mm³, the mice were anesthetized and then sacrificed by dislocation of the cervical spine.

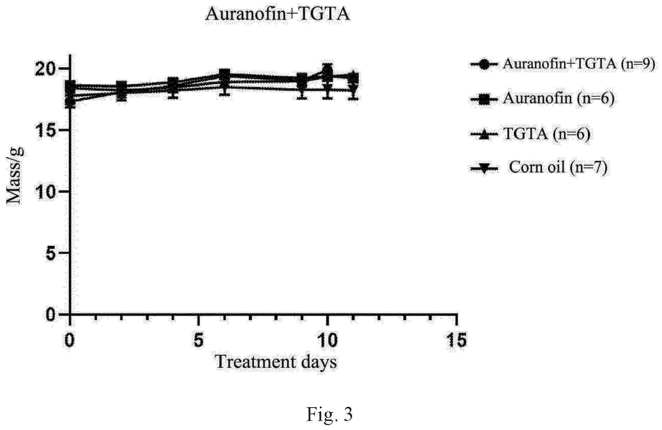

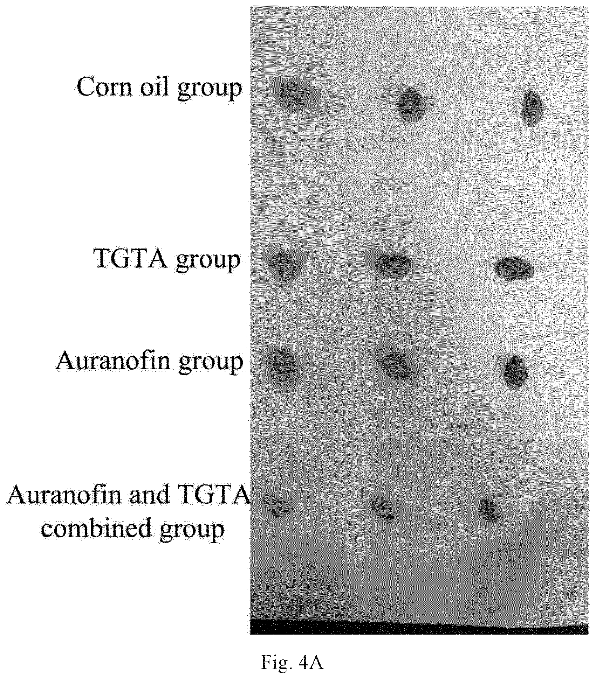

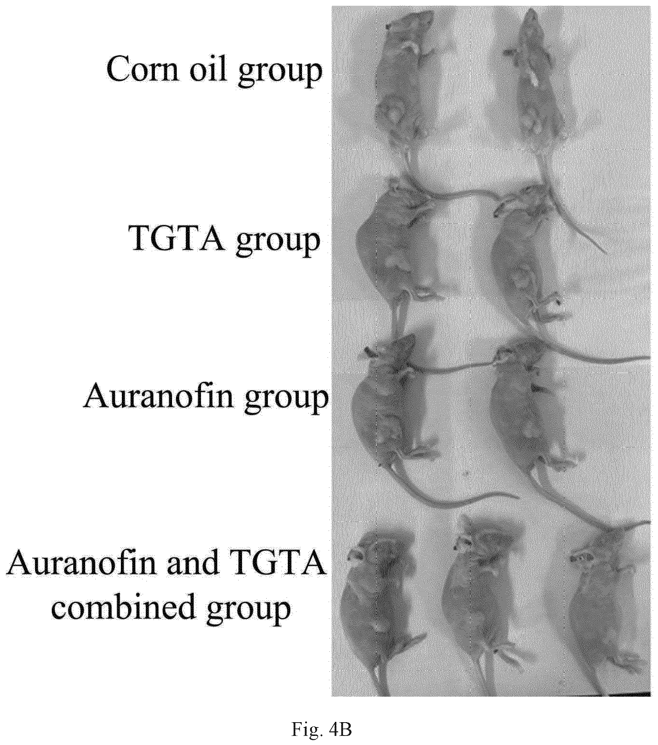

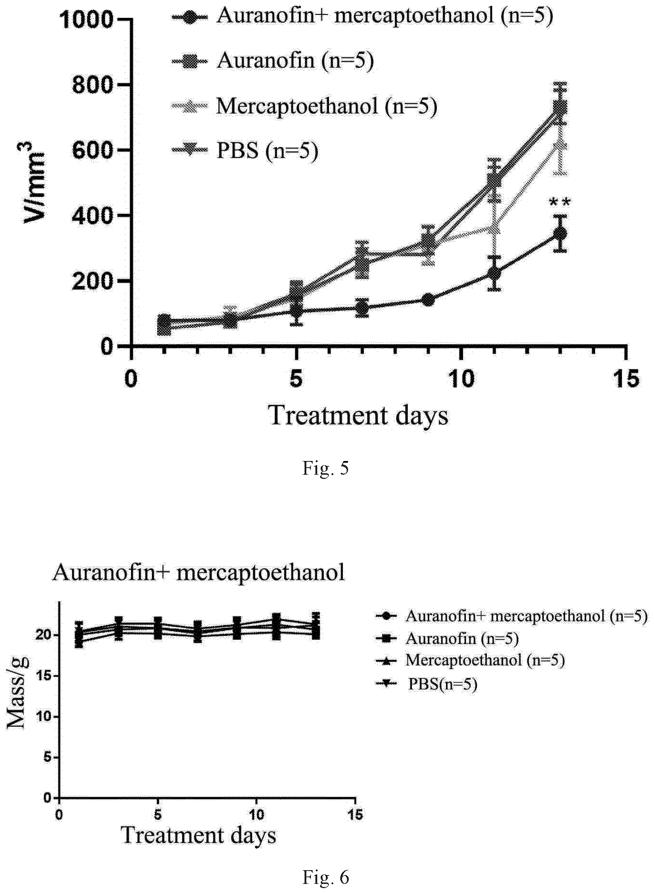

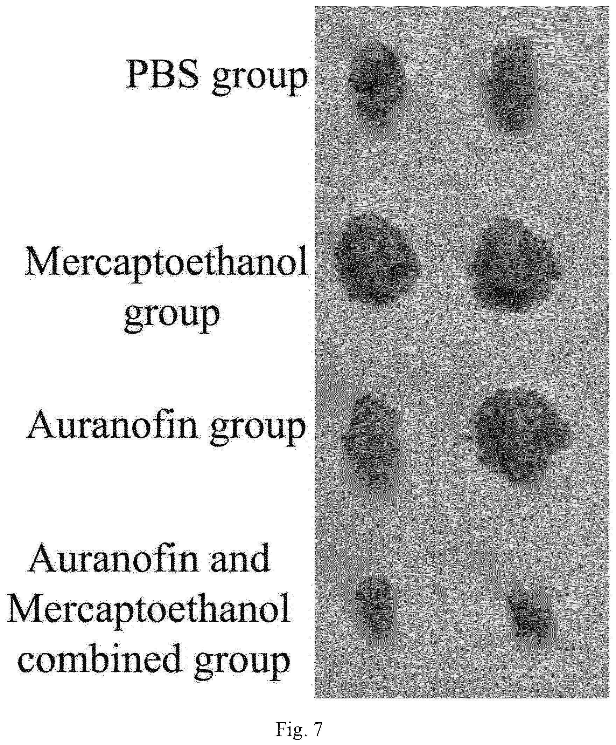

The results are shown in Figures 2-7.

The growth of tumors was not effectively inhibited in the control group, the auranofin alone group or the mercapto compound alone group. When auranofin and the mercapto compound were used in combination, the tumor growth was inhibited greatly.

The above embodiments are the optimal implementations of the present disclosure, which however are not limited by the embodiments, and any other changes, modifications, substitutions, combinations, simplifications made without deviating from the spirit and principle of the present disclosure shall be equivalent substitution methods, which all fall within in the protection scope of the present disclosure.

## Claims

1. A composition comprising auranofin and a mercapto compound.

2. The composition according to claim 1, wherein the mercapto compound comprises a compound with a structure as shown in Formula I or a thiolate:
R-SH (Formula I);
R is selected from any group;
the thiolate is preferably a metal thiolate; and
the mercapto compound is preferably one or more of β-mercaptoethanol, mercaptoethanamine, methylthioimidazole, propylthiouracil, dithiothreitol, N-acetylcysteine, glutathione, sodium thiosulfate, sodium thiophosphate, sodium diethyldithiocarbamate or 1-thio-β-D-glucose tetraacetate.

3. A drug comprising the composition according to claim 1 or claim 2.

4. A cell proliferation inhibitor comprising the composition of claim 1 or 2, wherein the cells preferably comprise colon cancer cells, liver cancer cells, lung cancer cells, gastric cancer cells, cervical cancer cells, and breast cancer cells.

5. Use of the composition according to claim 1 or claim 2 in the preparation of an antitumor drug.

6. The use according to claim 5, wherein the tumors comprise colon cancer, liver cancer, lung cancer, gastric cancer cells, cervical cancer cells, and breast cancer.

7. Use of the composition according to claim 1 or claim 2 in the preparation of a cell proliferation inhibitor, wherein the cells preferably comprise colon cancer cells, liver cancer cells, lung cancer cells, gastric cancer cells, cervical cancer cells and breast cancer cells.

8. Use of the composition according to claim 1 or claim 2 in the preparation of a TrxR inhibitor.

9. Use of a mercapto compound serving as a synergist of auranofin in the preparation of a TrxR inhibitor.

10. The use according to claim 9, wherein the mercapto compound comprises one or more of β-mercaptoethanol, mercaptoethanamine, methylthioimidazole, propylthiouracil, dithiothreitol, N-acetylcysteine, glutathione, sodium thiosulfate, sodium thiophosphate, sodium diethyldithiocarbamate or 1-thio-β-D-glucose tetraacetate.
